# EUROPEAN PATENT SPECIFICATION

(11) **EP 1 656 889 B1**
(45) Date of publication and mention of the grant of the patent: **11.08.2010**
(21) Application number: 05024863.2
(22) Date of filing: 14.11.2005
(51) Int. Cl.: A61B 10/00

(54) **Biopsy device with container**
Biopsiegerät mit Behälter
Outil de biopsie avec récipient

(30) Priority: 16.11.2004 JP 2004331840
(43) Date of publication of application: 17.05.2006
(73) Proprietor: Olympus Corporation, Tokyo 151-0072 (JP); OLYMPUS MEDICAL SYSTEMS CORP., Shibuya-ku Tokyo 151-0072 (JP)
(72) Inventor: Shibazaki, Tamaki Intell. Property Support Dept., Hachioji-shi Tokyo 192-8512 (JP); Sanuki, Hiromi Intellectual Property Support Dept., Hachioji-shi Tokyo 192-8512 (JP)
(74) Representative: von Hellfeld, Axel

(56) References cited:
- US-A- 5 554 151
- US-A- 5 573 008
- PATENT ABSTRACTS OF JAPAN vol. 1996, no. 09, 30 September 1996 (1996-09-30) & JP 08 117232 A (OLYMPUS OPTICAL CO LTD), 14 May 1996 (1996-05-14)

## Description

The present invention relates to a biopsy device containing a container designed to preserve a sample taken from a living body in a biopsy.

As a method of diagnosing a disease, the biopsy, which examines a living sample taken from a patient, is conventionally employed. As a device for obtaining a living sample at a minimally invasive approach, a biopsy needle, endoscopic biopsy forceps, endoscopic biopsy needle and the like are used.

An example of such an endoscopic biopsy needle is disclosed in Jpn. Pat. Appln. KOKAI Publication No. 8-117232. In order to obtain a living tissue using such an endoscopic biopsy needle, an endoscope is inserted to a body cavity. Then, the biopsy needle is put through the accessory channel of the endoscope and made to project from the distal end portion of the endoscope to reach an object part of the biopsy. While monitoring the object part of the biopsy using the endoscope, the operator resects and catches the living sample by operating the biopsy needle. After that, the operator withdraws the biopsy needle from the endoscope and transfers the catch living sample from the biopsy needle to a preservation container. Here, it is alternatively possible to use biopsy forceps in a similar manner in place of the biopsy needle.

On the other hand, Jpn. Pat. Appln. KOKAI Publication No. 2001-508674 discloses biopsy forceps designed to catch a living sample. The biopsy forceps disclosed here includes a narrow tube member provided along an endoscope. A pouring guide tube and a suction guide tube are buried in the tube member in such manners that the pouring guide tube extends from the proximal end portion to the distal end portion of the tube member, whereas the suction guide tube extends from the distal end portion to the proximal end portion. A pair of jaws are provided at the distal end portion of the tube member. When the pair of jaws are closed, the pouring guide tube and suction guide tube are coupled together in a liquid-tight manner. At the same time, the living sample held with teeth of the jaws are cut with the teeth and received inside of the closed jaws to be provided into the coupled guide tubes. The living sample provided into the guide tubes is carried by the liquid flowing from the pouring guide tube to the suction guide tube, to the proximal end portion of the suction guide tube. Then, the sample is collected from the liquid by a porous screen provided at the proximal end of the suction guide tube. Thus, the collected living sample is transferred to a preservation container.

The living sample collected and transferred to a preservation container with the device disclosed in Jpn. Pat. Appln. KOKAI Publication No. 8-117232 or Jpn. Pat. Appln. KOKAI Publication No. 2001-508674 is immersed in a sample preservation reagent or frozen by liquid nitrogen until test. It should be noted that the preservation method by means of liquid nitrogen is employed in, for example, the preservation of nucleic acid. As the method of testing a living sample, the pathologic observation of the tissue or the measurement of trace materials in a living sample, such as a certain antigen, RNA and DNA, is used for the diagnosis of diseases.

However, the method of catching a biopsy sample discussed in Jpn. Pat. Appln. KOKAI Publication No. 8-117232 or Jpn. Pat. Appln. KOKAI Publication No. 2001-508674 takes a long time to complete the preservation process of a living sample after cutting it from the living body. Further, the time required to complete the preservation varies greatly depending on the operator or the number of times of catch, etc. Therefore, the degree of the deterioration of the biopsy sample becomes larger and the dispersion of the degree of the deterioration becomes larger. As a result, the accuracy of the pathological test is degraded. This problem is particularly prominent in the test of easily deteriorating materials, such as the analysis of gene expression subjected to RNA.

Further, the method of catching a biopsy sample discussed in Jpn. Pat. Appln. KOKAI Publication No. 8-117232 or Jpn. Pat. Appln. KOKAI Publication No. 2001-508674 inevitably involves the transfer of a living sample from the biopsy device to the preservation container. During the transfer of a living sample, there is always a danger or possibility of contamination caused by dropping the sample from the container or mistaking a preservation container.

Therefore, the transfer of the sample must be carried out with the utmost caution, which makes the preservation process of the living sample into a container very complicated to carry out.

On the other hand, the method of storing a living sample by freezing it with liquid nitrogen can only be employed in limited facilities in which liquid nitrogen can be obtained. Further, it is necessary to maintain a living sample in an environment at about less than -80°C, for example, in the inside of the nitrogen liquid immediately after the freezing of the living sample, and therefore the operator is required to have an ability for the prompt measures. In addition, when the living sample is to be transported, it must be transported while the sample is maintained in dry ice or liquid nitrogen, and thus the transporter must pay the utmost attention to the control of the temperature of the sample, the prevention of the suffering from hypoxia, etc. Thus, in the above-described preservation method in which a living sample is stored by freezing it with liquid nitrogen, the handling of a stored living sample is very difficult.

Document US 5,573,008 concerns a device for collecting from a tissue surface a sample of tissue. The device includes a device body defining a forward-facing tissue receiving opening through which tissue may pass when the opening is near the tissue surface. A severing element is actuatable across the tissue receiving opening when tissue from the surface extends through the opening for severing the tissue from the surface. Furthermore, a storage space for storing the taken samples is provided.

The present invention has been achieved in consideration of the above-described drawbacks of the conventional techniques and the object thereof is to provide a biopsy device that includes a container, which can improve the accuracy of the pathological test and ease the preservation of a living sample into the container and the handling of a stored living sample.

Some or all of the above problems are overcome by a biopsy device having the features of claim 1.

According to an aspect of the present invention, there is provided a biopsy device characterized by comprising: an catching member that is able to catch and release a living sample; a sheath through which the catching member is able to be inserted; and a container that stores the living sample; wherein the container comprises: a container portion that is able to contain a sample preservation reagent and the living sample; a first removable insertion portion connected detachably and liquid-tightly to an end portion of the sheath, through which the catching member is able to be removably inserted from an outside of the container to the container portion; and a second removable insertion portion through which the catching member is able to be removably inserted from the container portion to an outside of the container in a liquid-tight manner.

The operation of this biopsy device is as follows. That is, the container is connected to an end of the sheath, and then the catching member is inserted through the sheath from the other end of the sheath to the above-mentioned end. Further, the catching member is inserted through the first removable insertion section, the container portion and the second removable insertion section to project from the container, and a living sample is caught with the catching member. After that, the catching member is pulled into the container, and inserted through the second removable insertion section to be located in the container portion. Subsequently, the living sample is released in the container portion to be stored in the sample preservation reagent in the container portion. Lastly, the container is removed from the sheath.

According to a preferred version of the first aspect of the present invention, there is provided a biopsy device of the first aspect, which is **characterized in that** the catching member is able to be removably inserted through the first removable insertion portion in a liquid-tight manner.

With this version of the biopsy device, the first and second removable insertion portions serve to enable the sealing of the sample preservation reagent in the container portion of the container.

According to another preferred version of the first aspect of the present invention, there is provided a biopsy device of the first aspect, which is characterized In that the first removable insertion portion communicates an inner cavity of the sheath to the container portion.

With this version of the biopsy device, the container is connected to one end of the sheath and the sample preservation reagent is transferred from the other end of the sheath to the one end, so as to be supplied to the container portion via the first removable insertion portion.

According to still another preferred version of the first aspect of the present invention, there is provided a biopsy device of the first aspect, which is **characterized in that** the container portion includes at least two sub-container portions divided from each other to store the sample preservation reagent and/or the living sample.

With this version of the biopsy device, each of the sub-container portions serves to contain the sample preservation reagent and/or living sample and the living sample is preserved in each of the sub-container portions.

According to still another preferred version of the first aspect of the present invention, there is provided a biopsy device of the first aspect, which is **characterized in that** said at least two sub-container portions are connected in series between the first removable insertion portion and the second removable insertion portion in such a manner that the catching member is inserted through in order, and the catching member is able to be removably inserted liquid-tightly through a connection portion provided between the sub-container portions.

The operation of this biopsy device is as follows. That is, a living sample is catch with the catching member projecting from the container and then the catching member is pulled into the container. After that, the catching member is positioned in a desired sub-container portion that is not located adjacent to the second removable insertion portion and the living sample is released there. Then, again, a living sample is catch with the catching member projecting from the container and the catching member is pulled into the container. After that, the catching member is positioned in a sub-container portion that is not located on the second removable insertion portion side with respect to the above desired sub-container portion and the living sample is released there.

According to still another preferred version of the first aspect of the present invention, there is provided a biopsy device of the first aspect, which is **characterized in that** the catching member includes a puncture needle.

With this version of the biopsy device, a living sample is taken with the puncture needle.

According to still another preferred version of the first aspect of the present invention, there is provided a biopsy device of the first aspect, which is **characterized in that** the catching member includes forceps.

With this version of the biopsy device, a living sample is taken with the forceps.

According to still another preferred version of the first aspect of the present invention, there is provided a biopsy device of the first aspect, which is **characterized in that** the container includes an identification tag.

With this version of the biopsy device, a container is identified by the identification tag.

According to still another preferred version of the first aspect of the present invention, there is provided a biopsy device of the first aspect, which is **characterized in that** the sample preservation reagent includes a nucleic acid decomposition inhibiter reagent.

With this version of the biopsy device, nucleic acid is preserved by the nucleic acid decomposition inhibiter reagent.

According to still another preferred version of the first aspect of the present invention, there is provided a biopsy device of the first aspect, which is **characterized in that** the sample preservation reagent includes a cell preservation reagent.

With this version of the biopsy device, cell is preserved by the cell preservation reagent.

According to still another preferred version of the first aspect of the present invention, there is provided a biopsy device of the first aspect, which is **characterized in that** at least two of the sub-container portions contain different types of sample preservation reagents from each other.

With this version of the biopsy device, each of at least two of the sub-container portions that contain different types of sample preservation reagents from each other, preserves a respective living sample of different types, in accordance with the type of the sample preservation reagent.

According to the present invention, the accuracy of the pathological test can be improved, and the preservation of a living sample in the container and the handling of the preserved living sample can be facilitated.

This summary of the invention does not necessarily describe all necessary features so that the invention may also be a sub-combination of these described features.

The invention can be more fully understood from the following detailed description when taken in conjunction with the accompanying drawings, in which:
FIG. 1 is a diagram showing a perspective view of an endoscope apparatus including a biopsy device according to the first embodiment of the present invention;
FIG. 2A is a diagram showing a longitudinal cross section of a biopsy device according to the first embodiment of the present invention;
FIG. 2B is a diagram showing a longitudinal cross section of a container for a biopsy device according to the first embodiment of the present invention;
FIG. 3 is a diagram showing a longitudinal cross section of a biopsy device according to the second embodiment of the present invention;
FIG. 4 is a diagram showing a longitudinal cross section of a biopsy device according to the third embodiment of the present invention; and
FIG. 5 is a diagram showing a perspective view of an endoscope apparatus including a biopsy device according to the fourth embodiment of the present invention.

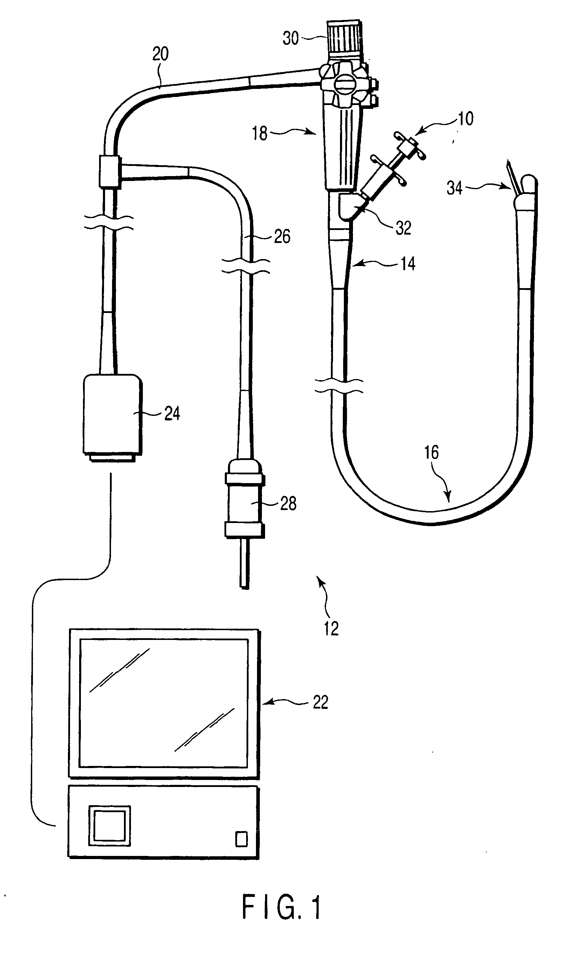

The first embodiment of the present invention will now be described with reference to FIGS. 1 to 2B. FIG. 1 shows a brief structure of an entirety of an endoscope apparatus 12 including a biopsy device 10 according to the first embodiment of the present invention. The endoscope apparatus 12 includes an ultrasonic endoscope 14 that carries out the observation by utilizing the ultrasonic wave. The ultrasonic endoscope 14 includes a slim insertion section 16 which is inserted to a body cavity. A operation section 18, which is grasped by the operator to operate, is provided at a proximal end portion of the insertion section 16. A universal cord 20 is extended from a side portion of the operation section 18, and an ultrasonic connector 24, which is to be connected to an ultrasonic image observation device 22, is provided at an extended end of the universal cord 20. Further, a light source cable 26 is branched off from a middle portion of the universal cord 20, and a light source connector 28, which is connected to a light source device (not shown in the figures), is provided at an extended end of the light source cable 26.

Here, an ultrasonic probe that can visualize an inside of a living tissue is provided at a distal end portion of the insertion section 16. A signal line that can transmit image signals is extended from the ultrasonic probe. The signal line is provided through the insertion section 16, the operation section 18 and the universal cord 20 to be connected to the ultrasonic connector 24. Thus, image signals are output to the ultrasonic image observation device 22 via the ultrasonic connector 24.

In addition, an illumination optical system for illuminating an object to be observed and an observation optical system for observing the object are provided at a distal end of the insertion section 16. A distal end of a light guide used to guide the illumination light is connected to the illumination optical system. The light guide is provided through the insertion section 16, the operation section 18, the universal cord 20 and the light source cable 26 to be connected to the light source connector 28. Thus, illumination light is supplied to the light guide from the light source device via the light source connector 28. On the other hand, a distal end of an image guide used to transmit the observation image is connected to the illumination optical system. The light guide is provided through the insertion section 16 and the operation section 18 to be connected to an eyepiece section 30 provided in the operation section 18.

An accessory channel 10, in which the biopsy device 10 is put, extends within the insertion section 16. A channel opening 32, which is an entrance of the accessory channel is formed in the operation section 18, and an accessory opening 34, which is an exit, is formed in the distal end portion of the insertion section 16. It should be noted that in this embodiment, an elevating lever used to adjust the projecting direction of the distal end portion of the biopsy device 10 is provided at the accessory channel.

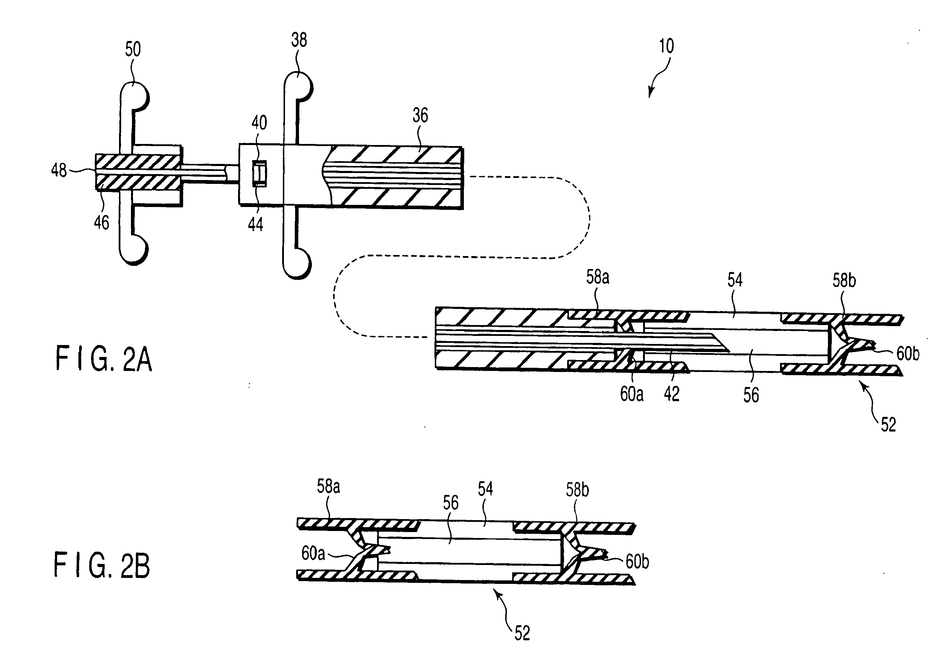

As shown in FIG. 2A, the biopsy device 10 has a slim cylindrical soft sheath 36 to be inserted to the accessory channel. A first grip 38, which is held by the operator, is provided to project from the outer circumferential surface of a proximal end portion of the sheath. A slit-like positioning line confirmation window 40 is made in the proximal end portion of the sheath 36, and through the window, a positioning line 44 for confirming the position of a puncture needle 42 can be observed.

The puncture needle 42 is inserted to the cavity of the sheath 36, and the needle 42 serves as an catching member for catching a living sample. In this embodiment, a hard aspiration needle is used as the puncture needle 42. It should be noted that a suction needle usually has a needle diameter of 22G to 25G, and is used in puncture aspiration of a tumor, lymph node cell, etc. A catch living sample can be subjected to a gene analysis, cytodiagnosis or the like. An aspiration needle has a narrow needle diameter, and therefore the catch of a living sample with use of an aspiration needle involves a very low invasion, which requires a minimum amount of anesthesia. Thus, the burden on patients can be reduced.

The tip end of the puncture needle 42 is formed to be removably inserted in the distal opening of the sheath 36. On the other hand, the positioning line 44 is provided at the proximal end of the puncture needle 42. The positioning line is designed for the operator to be able to confirm the position of the tip end of the puncture needle 42 on the handling side. As the puncture needle 42 is moved forwards and backwards in relative to the sheath 36 to align the positioning line 44 with the positioning line confirmation window 40, the tip end of the puncture needle 42 is located inside a container 52, which will be described later. Further, a soft tube 46 is connected to the proximal end of the puncture needle 42. The tube 46 has an outer diameter larger than that of the puncture needle 42 and an inner diameter substantially equal to that of the puncture needle 42. With this structure, the inner cavity of the puncture needle 42 is connected through to the inner cavity of the tube 46. A proximal opening of the tube 46 serves as a connection opening 48 to which a syringe is connected. The tube 46 and the puncture needle 42 make a connecting path that communicates the connection opening 48 to the distal opening of the puncture needle 42. It should be noted here that the measurements and structure of the connection opening 48 are selected from various designs in accordance with a desired syringe. Further, a second grip 50, which is held by the operator, is provided to project from the outer circumferential surface of the proximal end portion of the tube 46.

The container 52, which is used to store a living sample, is removably mounted to the distal end portion of the sheath 36. The container 52 has a cylindrical shape that is coaxial with the sheath 36 and has substantially the same outer diameter as that of the sheath 36. The puncture needle 42 projecting from the distal opening of the sheath 36 is inserted through the container in a forward-and-backward movable manner. It should be noted that the container 52 should preferably be formed of an elastic material so that it is inserted easily through the accessory channel of the ultrasonic endoscope 14 (see FIG. 1).

In more detail, the container 52 includes a cylindrical member 54 which makes a central part of the container 52, and an inner cavity of the cylindrical member 54 forms a container portion 56 for storing a sample preservation reagent and a living sample. A narrow diameter portion is formed in a proximal end of the cylindrical member 54, and a distal end side of a first rubber tube 58a is liquid-tightly fit with an outer circumferential surface of the narrow diameter portion. A narrow diameter portion is formed in the distal end of the sheath 36 as well, and the other end side of the first rubber tube 58a is liquid-tightly fit with an outer circumferential surface of this narrow diameter portion. A first valve member 60a is formed at a central part of the inner cavity of the first rubber tube 58a. The puncture needle 42 projecting from the distal opening of the sheath 36 is removably inserted liquid-tightly through the first valve member 60a to the container portion 56. The first valve member 60a further has a function of maintaining the container portion 56 in a liquid-tight manner from the outside of the container 52. As described above, in this embodiment, the first rubber tube 58a forms a first removable insertion portion that is detachably and liquid-tightly connected to one end of the sheath 36 and through which the catching member is able to be removably inserted from outside of the container 52 to the container portion 56 in a liquid-tight manner.

On the other hand, a narrow diameter portion is formed in a distal end of the cylindrical member 54 as well, and a proximal end side of a second rubber tube 58b is liquid-tightly fit with an outer circumferential surface of this narrow diameter portion. A second valve member 60b is formed at a central part of the inner cavity of the second rubber tube 58b. The puncture needle 42 projecting from the distal opening of the cylindrical member 54 is removably inserted liquid-tightly through the second valve member 60b to the outside of the container. The second valve member 60b further has a function of maintaining the container portion 56 in a liquid-tight manner from the outside of the container 52. With this structure, the container portion 56 is liquid-tightly closed by the first and second valves 60a and 60b. As described above, in this embodiment, the second rubber tube 58b forms a second removable insertion portion through which the catching member is able to be removably inserted from the container portion 56 to the outside of the container 52 in a liquid-tight manner.

In this embodiment, the first rubber tube 58a and the second rubber tube 58b have the same structure. More specifically, the first and second rubber tubes 58a and 58b each have a proximal end side that is formed liquid-tightly fittable with the distal end of the sheath 36 or the distal end of the cylindrical member 54, respectively, and a distal end side that is formed liquid-tightly fittable with the proximal end of the cylindrical member 54. Further, the tubes respectively have valve members 60a and 60b provided at the central portion of the inner cavity. The puncture needle 42 is able to be removably inserted through valve members 60a and 60b from the proximal end side to the distal end side in a liquid-tight manner.

The container portion 56 is filled with a sample preservation reagent that can prevent the deterioration of the living sample and sealed. For example, nucleic acid is decomposed by a nuclease such as DNasel, which is present in the environment or a living body. When nucleic acid is selected as the living sample, an medicine that can block the activity of the nuclease, such as nuclease inhibitor, ribonuclease inhibitor, deoxyribonuclease inhibitor or reagent composed of surfactant can be used. Alternatively, a commercially available nucleic acid decomposition inhibiter reagent such as RNAlater (registered trademark) of Ambion Co. Ltd. can be used.

It should be noted that the first rubber tube 58a is detachably attached to the distal end of the sheath 36, and therefore the container 52 (see FIG. 2B) is detachably attached to the distal end of the sheath 36. The container 52 can be removed from the sheath 36 and directly loaded to the preservation device so as to be stored.

Next, the operation of the biopsy device 10 of this embodiment will now be described step by step.

### Step 1-1

After a sample preservation reagent is sealed in the container portion 56 of the container 52, the container 52 is mounted to the distal end portion of the sheath 36. In this embodiment, a nucleic acid decomposition inhibiter reagent is used as the sample preservation reagent.

### Step 1-2

The puncture needle 42 is put through the sheath 36 in advance, and the second grip 50 is pushed to forward with respect to the first grip 38 until the positioning line 44 is aligned with the positioning line confirmation window 40. Thus, the tip end of the puncture needle 42 is made to project from the distal opening of the sheath 36, and then inserted to the container portion 56 through the first valve member 60a of the container in a liquid-tight manner, so as to be located inside the container portion 56.

### Step 1-3

The insertion section 16 of the ultrasonic endoscope 14 is inserted to the body cavity of a subject patient, and the distal end of the insertion section 16 is operated to approach the diseased part, which is the biopsy object part. Then, with use of the ultrasonic probe, the region of the diseased part is visualized as an ultrasonic image on the ultrasonic image observation device 22.

### Step 1-4

The biopsy device 10 is inserted from the channel opening 32 of the ultrasonic endoscope 14, and the biopsy device 10 is pushed to forward with respect to the ultrasonic endoscope 14 within the accessory channel to project the tip end of the biopsy device 10 from the accessory opening 34 formed at the distal end of the insertion section 16. At the same time, the elevating lever is operated to orient the projecting direction of the distal end of the biopsy device 10 towards the diseased part.

### Step 1-5

The second grip 50 is pushed forward with respect to the first grip 38 and thus the puncture needle 42 is pushed forward with respect to the sheath 36. The puncture needle 42 is inserted through the second valve member 60b of the container 52 liquid-tightly and made to project from the container portion 56 to the outside of the container 52.

### Step 1-6

While observing with the endoscope 14, the second grip 50 is operated with respect to the first grip 38 to insert the puncture needle 42 to the diseased part. Then, while observing the ultrasonic image on the ultrasonic image observation device 22, the tip end of the puncture needle 42 is made to reach the target part within the diseased part.

### Step 1-7

The living sample is catch with the puncture needle 42. More specifically, a syringe is connected to the connection opening 48 and the inner cylinder of the syringe is pulled to suction the inner cavity of the puncture needle 42. Thus, the living sample of the diseased part is contained in the inner cavity of the puncture needle 42.

### Step 1-8

The second grip 50 is pulled with respect to the first grip 38 until the positioning line 44 is aligned with the positioning line confirmation window 40. In this manner, the puncture needle 42 is pulled with respect to the sheath 36 so as to be inserted through the second valve member 60b liquid-tightly. Then, the tip end of the puncture needle 42 is placed in the container portion 56 of the container 52.

### Step 1-9

The inner cylinder of the syringe connected to the connection opening 48 is pushed to discharge the living sample of the diseased part contained in the inner cavity of the puncture needle 42 to the container portion 56, and thus the living sample is immersed in the sample preservation reagent. In this embodiment, a nucleic acid decomposition inhibiter reagent is used as the sample preservation reagent, and therefore the decomposition of the nucleic acid can be suppressed.

### Step 1-10

The second grip 50 is pulled with respect to the first grip 38 so as to insert the puncture needle 42 through the first valve member 60a liquid-tightly. Thus, the puncture needle 42 is withdrawn from the container portion 56 so as to be located inside the sheath 36.

### Step 1-11

The biopsy device 10 is pulled with respect to the ultrasonic endoscope 14 so as to be pulled out from the ultrasonic endoscope 14.

### Step 1-12

The container 52 mounted in the sheath 36 is removed. Thus removed container is stored directly in the preservation device and thus the living sample is preserved until the test.

### Step 1-13

In the case where the sampling is carried out at a plurality of sites, Steps 1-1 to 1-12 are repeated accordingly.

With the above-described procedure, the biopsy device 10 of this embodiment exhibits the following effects. That is, a living sample is contained in the inner cavity of the puncture needle 42 and then the puncture needle 42 is placed in the container portion 56. After that, the living sample is discharged from the puncture needle 42 to the sample preservation reagent filled in the container portion 56, and thus the preservation of the living sample in the container 52 is completed. As described, the time required from the catch of a living sample to the preservation can be shortened and made substantially constant. Therefore, the degree of deterioration of the living sample can be lowered and the variation in the degree of deterioration from one sample to another can be suppressed. Thus, the accuracy of the pathological test can be improved. Further, unlike the conventional technique, the transfer of a living sample from the biopsy device to the preservation container is no longer required in the present invention. Thus, it is possible to prevent a contamination caused by dropping the sample while transferring it and mistaking a preservation container to be transferred. Thus, the present invention can facilitate the preservation of a living sample into a container. Furthermore, a sample preservation reagent is used to preserve living samples, and therefore they can be preserved at room temperature, which makes the preservation and transport of the container 52 easily. Thus, the preservation of living samples can be very much facilitated.

The first alternative version of the first embodiment of the present invention will now be described. In the first embodiment described above, a hard aspiration needle is used; however it is alternatively possible to use such a model that has a hard tip end portion insertable to a living body and capable of catching and holding a living sample within its inner cavity, to which a tube-like soft member is connected. Alternatively, a different type of puncture needle, such as sampling needle can be used in place of the puncture needle 42. A usual sampling needle has a needle diameter of about 16G, and catch living samples with such a sampling needle can be used in gene analysis, tissue diagnosis, and the like. The sampling needle has a large needle diameter and therefore it can catch a living sample while maintaining the structure of its tissue. Therefore, with use of such a needle, a more accurate diagnosis can be expected. A type of puncture needle should be selected in accordance with the object of clinical test, and various types of puncture needles can be employed.

The second alternative version of the first embodiment of the present invention will now be described. In the first embodiment described above, the puncture needle 42 is used as a member for catching living samples; however it is alternatively possible to use forceps that is able to catch and release a living sample as it is closed and opened. In the case where a living sample is catch with forceps, they are made to project from the container 52, and then are closed to cut the living sample and hold it. After that, while closing the forceps, they are withdrawn into the container portion 56 and then are opened to release the living sample in the container portion 56. With use of the puncture needle 42, it is possible to catch a living sample from an inside of a diseased part, whereas with use of the forceps, it is possible to catch a living sample from the surface of a diseased part.

The third alternative version of the first embodiment of the present invention will now be described. In the first embodiment described above, a nucleic acid decomposition inhibiter reagent is used as the sample preservation reagent, it is alternatively possible to use a tissue preservation reagent such as formaline, paraformaldehyde or acetone. In the case where a living sample is immersed in such a tissue preservation reagent, the form of cells can be preserved and therefore the pathologic test of tissues can be carried out.

The fourth alternative version of the first embodiment of the present invention will now be described. In the first embodiment described above, the valve mechanism of the first and second rubber tubes 58a and 58b is used to insert and remove the puncture needle 42 in a liquid-tight manner. In place, a rubber film can be employed, and this film should be of such a type that can maintain the liquid-tightness with respect to the puncture needle 42 when the needle is inserted therethrough and further maintain the inside of the container portion 56 in a liquid-tight manner by self-closing the punctured hole when the puncture needle 42 is withdrawn.

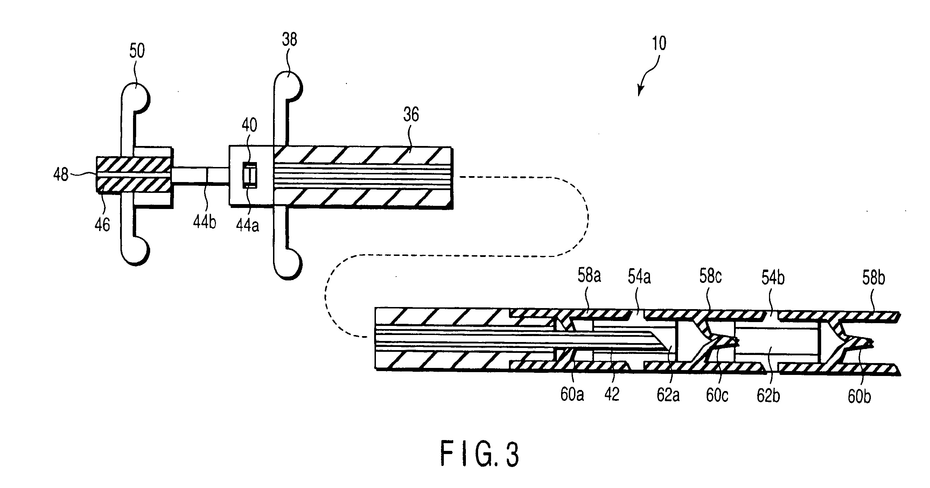

FIG. 3 shows the second embodiment of the present invention. In this embodiment, structural members that have the same function as those of the first embodiment will be designated by the same reference numerals and the explanations therefore will be omitted. In this embodiment, first and second sub-container portions 62a and 62b are provided as container portions for storing a sample preservation reagent and/or a living sample.

The container 52 of this embodiment is made of first and second cylindrical members 54a and 54b, which are similar to the cylindrical member 54 (see FIGS. 2A and 2B) of the first embodiment, and first to third rubber tubes 58a, 58b and 58c, which are similar to the first and second rubber tubes 58a and 58b (see FIGS. 2A and 2B) of the first embodiment. In more detail, a distal end side of the first rubber tube 58a is liquid-tightly fit with a proximal end portion of the first cylindrical member 54a, and a proximal end side of the third rubber tube 58c is liquid-tightly fit with a distal end portion of the first cylindrical member 54a. Further, a distal end side of the third rubber tube 58c is liquid-tightly fit with a proximal end portion of the second cylindrical member 54b, and a proximal end side of the second rubber tube 58b is liquid-tightly fit with a distal end portion of the second cylindrical member 54b.

An inner cavity of the first cylindrical member 54a forms the first sub-container portion 62a, and an inner cavity of the second cylindrical member 54b forms the second sub-container portion 62a. Through a third valve member 60c of the third rubber tube 58c, a puncture needle 42 is able to be removably inserted from the first sub-container portion 62a to the second sub-container portion 62b in a liquid-tight manner. The third valve member 60c has a function of maintaining the first sub-container portion 62a and the second sub-container portion 62b liquid-tightly with respect to each other. Thus, the first sub-container portion 62a is liquid-tightly closed by the first and third valve members 60a and 60c, and the second sub-container portion 62b is liquid-tightly closed by the second and third valve members 60b and 60c. In this embodiment, as the sample preservation reagent, a nucleic acid decomposition inhibiter reagent is sealed in the first and second sub-container portions 62a and 62b.

First and second positioning lines 44a and 44b are provided in a proximal end portion of the puncture needle 42. The first positioning line 44a is located on a distal end side with respect to the second positioning line 44b. When the first positioning line 44a can be observed in a positioning line confirmation window 40, the tip end of the puncture needle 42 is located in the first sub-container portion 62a, whereas when the second positioning line 44b can be observed in the positioning line confirmation window 40, the tip end of the puncture needle 42 is located in the second sub-container portion 62a.

Next, the operation of the biopsy device 10 of this embodiment will now be described step by step. Steps 2-1, 2-2, ... 2-6 and 2-7 of this embodiment, which are carried out to catch a first living sample, are similar to Steps 1-1, 1-2, ... 1-6 and 1-7 of the first embodiment, and therefore the explanations for these steps will be omitted. The following steps will now be described.

### Step 2-8

The second grip 50 is pulled with respect to the first grip 38 until the first positioning line 44a is aligned with the positioning line confirmation window 40. In this manner, the tip end of the puncture needle 42 is placed in the first sub-container portion 62a.

### Step 2-9

The first living sample of the diseased part contained in the inner cavity of the puncture needle 42 is discharged to the first sub-container portion 62a, and thus the first living sample is immersed in the sample preservation reagent.

### Step 2-10

The second grip 50 is pushed forward with respect to the first grip 38 so as to insert the tip end of the puncture needle 42 through the third valve member 60c of the container 52 liquid-tightly from the first sub-container portion 62a to the second sub-container portion 62b. After that, the tip end of the puncture needle 42 is inserted through the second valve member 60b liquid-tightly, and made to project from the second sub-container portion 62b to the outside of the container 52.

### Steps 2-11 and 2-12

As described in Steps 1-6 and 1-7 of the first embodiment, the second living sample is catch in the inner cavity of the puncture needle 42.

### Step 2-13

The second grip 50 is pulled with respect to the first grip 38 until the second positioning line 44a is aligned with the positioning line confirmation window 40. In this manner, the tip end of the puncture needle 42 is placed in the second sub-container portion 62a.

### Step 2-14

The second living sample of the diseased part contained in the inner cavity of the puncture needle 42 is discharged to the second sub-container portion 62b, and thus the second living sample is immersed in the sample preservation reagent.

### Step 2-15

The second grip 50 is pulled with respect to the first grip 38 so as to insert the tip end of the puncture needle 42 through the third valve member 60c liquid-tightly, and further withdraw it from the first sub-container portion 62a. After that, the tip end of the puncture needle 42 is inserted through the first valve member 60a liquid-tightly, and further withdrawn from the first sub-container portion 62a to be located inside the sheath 36.

### Steps 2-16 and 2-17

As described in Steps 1-11 and 1-12 of the first embodiment, the biopsy device 10 is pulled out from the ultrasonic endoscope 14 and the container 52 mounted in the sheath 36 is removed. Thus removed container 52 is stored directly in the preservation device.

### Step 2-18

In the case where the sampling is carried out at a plurality of parts, Steps 2-1 to 2-17 are repeated accordingly.

With the above-described steps, the biopsy device 10 of the embodiment exhibits the following advantages. That is, the container 52 includes a plurality of sub-container portions 62a and 62b that can store a living sample and/or a sample preservation reagent. With this structure, it is possible to take living samples of a plurality of parts by one insertion of the biopsy device 10.

The first alternative version of the second embodiment of the present invention will now be described. In the second embodiment, the first and second sub-container portions 62a and 62b are used as the plurality of sub-container portion; however it is alternatively possible to prepare more than two sub-container portions. In such a case, further more living samples can be taken by one insertion of the biopsy device 10. Further, as in the first and second embodiments, in the case where a plurality of rubber tubes having the same structure and a plurality of cylindrical members having the same structure are employed, a container including a desired number of sub-container portions can be easily prepared by connecting the rubber tubes and cylindrical members successively in series.

The second alternative version of the second embodiment of the present invention will now be described. In the second embodiment and its alternative version, the same type of sample preservation reagent (nucleic acid decomposition inhibiter reagent) is sealed in each of the independent sub-container portions. In place, different types of sample preservation reagents can be sealed in the sub-container portions. For example, a nucleic acid decomposition inhibiter reagent can be sealed in some of the sub-container portions, whereas a tissue preservation reagent can be sealed in the rest of the sub-container portions. In such a case, the catch of a plurality of living sample to be subjected to nucleic acid test and the catch of a plurality of living sample to be subjected to tissue test can be carried out by inserting the biopsy device 10 only one time.

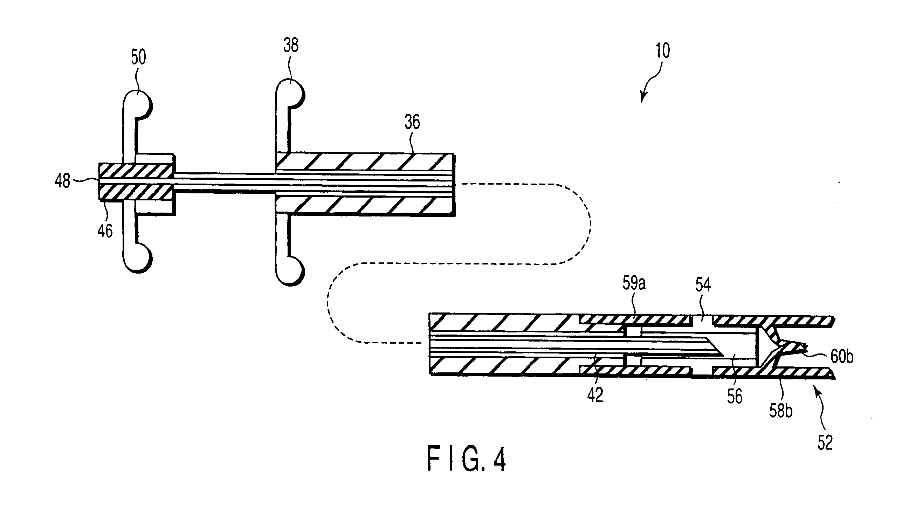

FIG. 4 shows the third embodiment of the present invention. In this embodiment, structural members that have the same function as those of the first embodiment will be designated by the same reference numerals and the explanations therefore will be omitted. In this embodiment, the first valve portion 60a (see FIGS. 2A and 2B) is not provided in the first rubber tube 59a, and the inner cavity of the sheath 36 and the container portion 56 are communicated with each other. Even if the puncture needle 42 is inserted through the sheath 36, the container portion 56 and the proximal opening of the sheath 36 are communicated to each other due to the clearance between the inner circumferential surface of the sheath 36 and the outer circumferential surface of the puncture needle 42.

Next, the operation of the biopsy device 10 of this embodiment will now be described step by step.

### Step 3-1

The container is mounted to the distal end portion of the sheath 36 without sealing a sample preservation reagent in the container portion 56 of the container 52.

### Steps 3-2 to 3-8

As described in Steps 1-2 and 1-8 of the first embodiment, a living sample is catch in the inner cavity of the puncture needle 42, and the distal end of the puncture needle 42 is placed inside the container portion 56.

### Step 3-9

An empty syringe is connected to the connection opening 48 and the inner cylinder of the syringe connected to the connection opening 48 is pushed to discharge the living sample of the diseased part contained in the inner cavity of the puncture needle 42 to the container portion 56.

### Step 3-10

A syringe that sucks and contains a sample preservation reagent is connected to the connection opening 48 and the inner cylinder of the syringe is pushed to inject the sample preservation reagent to the container portion 56 via the inner cavity of the puncture needle 42. Thus, the living sample is immersed in the sample preservation reagent. During this operation, the air inside the container portion 56 is released from the proximal opening of the sheath 36 via a gap between the inner circumferential surface of the sheath 36 and the outer circumferential surface of the puncture needle 42.

### Steps 3-11 and 3-13

As described in Steps 1-10 and 1-12 of the first embodiment, the tip end of the puncture needle 42 is pulled out from the container portion 56 so as to be located inside the sheath 36. Then, the biopsy device 10 is pulled out from the ultrasonic endoscope 14 and the container 52 mounted in the sheath 36 is removed.

### Step 3-14

In the case where the sampling is carried out at a plurality of parts, Steps 3-1 to 3-13 are repeated accordingly.

With the above-described steps, the biopsy device 10 of the embodiment exhibits the following advantages. That is, after catching a living sample, the puncture needle 42 is placed in the container 52, and then the sample preservation reagent is injected to the container portion 56. So, it can be avoided that the puncture needle 42 is inserted or removed between the container portion 56 and the outside of the container 52 while the sample preservation reagent is sealed in the container 52. Therefore, the possibility of leaking the sample preservation reagent from the container 52 to the body cavity can be lowered.

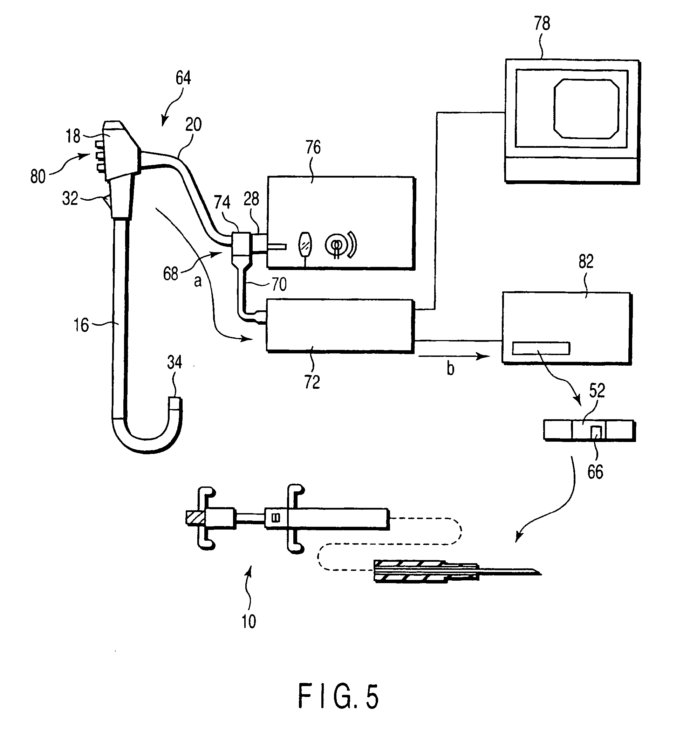

FIG. 5 shows the fourth embodiment of the present invention. In this embodiment, structural members that have the same function as those of the first embodiment will be designated by the same reference numerals and the explanations therefore will be omitted. In this embodiment, the biopsy device 10 of the first embodiment is combined with an electronic endoscope 64, and an identification tag 66 for identifying a container 52 is provided for each of the containers 52.

As shown in FIG. 5, the electronic endoscope 64 of the embodiment has an insertion section and a operation section 18 as in the first embodiment. A universal cord 20 is extended from a side portion of the operation section 18, and an endoscope connector 68 is provided in a proximal end portion of the universal cord 20. The endoscope connector 68 is made of a light source connecter 28 connected to a light source device 76 and an electric connector 74 connected to a video system center 72 via a connection cord 70.

Here, as in the first embodiment, an illumination optical system is provided in the distal end portion of the insertion section 16, and illumination light is supplied from the light source device 76 to the illumination optical system via a light guide. Further, a CCD is provided on the distal end portion of the insertion section 16 in order to pick up an observation image, and a signal line that transmit image signals is extended from the CCD. The signal line is put through the insertion section 16, the operation section 18 and the universal cord 20 and then connected to the electric connector 74, and the image signals are output to the video system center 72 via the electric connector 74 and the connection cord 70. The video system center 72 processes the input image signals and displays the observation image on a monitor 78. Further, as in the first embodiment, an accessory channel is extended in the insertion section 16, a channel opening 32 is provided in the operation section 18, and an accessory opening 34 is provided in the distal end of the insertion section 16.

The operation section 18 has a button 80 for outputting an unloading instruction signal a that instructs unloading of a container 52. The signal line is extended from the button 80 and put through the universal cord 20 to be connected to the electric connector 74. With this structure, the unloading instruction signal a is output to the video system center 72 via the electric connector 74 and the connection cord 70. When the unloading instruction signal a is input thereto, the video system center 72 outputs a container unloading instruction signal b to a container stocker 82 that stocks a plurality of containers 52. When the container unloading instruction signal b is input thereto, the container stocker 82 unloads the containers 52, and reads the identification tag 66 of each container 52. As the identification tag 66, for example, a bar cord, a microchip or an IC tag is employed. The container stocker outputs read identification data to the video system center 72.

When the identification data is input thereto, the video system center 72 establishes associations between various types of data in the biopsy and the identification data, and stores them. For example, as to a certain container 52, it is possible to associate the identification data of the container 52 to the observation image data stored during the operation of the biopsy. Alternatively, as to a certain container 52, it is possible to specify the location on the observation image where the living sample is to be catch in the biopsy and associate the identification data of the container 52 to the catch location data. Further, as to a certain container 52, it is possible to associate the identification data of the container 52 to the patient data, sampling time and date data, etc.

Next, the operation of the biopsy device 10 of this embodiment will now be described step by step.

### Step 4-1

The electronic endoscope 64 is inserted to a body cavity and the distal end of the insertion section 16 is made to approach the diseased part, which is the object part to be biopseid. Then, the region of the diseased part is set within the field of view of the electronic endoscope 64 and the observation image is displayed on the monitor 78.

### Step 4-2

The button 80 of the operation section 18 is operated to output an unloading instruction signal a to the video system center 72, and thus a container unloading instruction signal b is output to a container stocker 82 from the video system center 72. The container stocker 82 unloads not-yet-used containers 52, and reads the identification tag 66 of each of these containers 52, to output the read identification data to the video system center 72.

### Step 4-3

A container 52 unloaded from the container stocker 82 is mounted to the distal end of the sheath 36 of the biopsy device 10, and then a biopsy sample is taken and preserved in the same manner as discussed in the first embodiment. During this operation, the video system center 72 establishes associations between various types of data in the biopsy and the identification data, and stores them. In this embodiment, the location where the living sample is to be catch in the biopsy is specified on the observation image. Then, the video system center 72 adds the sampling time and date data and the pre-stored patient data to the catch location data, and associates the set of these data with the identification data of the container 52 to be stored.

### Step 4-4

When the living sample is tested later, the identification data are read from the identification tag of the container 52 in which the living sample is stored, and the living sample catch location, sampling date and time and the patient are referred to from the read identification data. With reference to these associated data, the diagnosis of the disease is carried out.

As described above, the biopsy device 10 of this embodiment exhibits the following advantage. The container stocker 82 unloads not-yet-used containers 52, and reads the identification tag 66 of each of these containers 52, to output the read identification data to the video system center 72. Then, the video system center 72 establishes associations between various types of data in the biopsy and the identification data, and stores them. When the living sample is tested later, various types of data in the biopsy are referred to from the read identification data of the container 52 in which the living sample is stored, and with reference to these associated data, the diagnosis of the disease can be carried out. This makes it possible the accuracy of the diagnosis. Particularly, in the treatment of cancers or the like, where the process observation after the treatment of the diseased part is carried out, the above-described technique is particularly effective in order to check the change in state over time after the treatment of the diseased part.

In this embodiment, the button 80 for outputting an unloading instruction signal is provided in the operation section 18 of the endoscope; however it is alternatively possible to provide this button 80 at an arbitrary location. For example, it may be provided in the video system center 72 or the container stocker 82.

It should be noted that the operation steps in all of the embodiments described above are only examples, and they can be modified appropriately in accordance with necessity.

## Claims

1. A biopsy device (10) comprising:
a catching member (42) that is adapted to catch and release a biopsy sample;
a sheath (36) through which the catching member is insertable; and
a container (52) **characterized in that** the container is detachably attached to the distal end of the sheath (36) for storing the biopsy sample;
wherein the container (52) comprises:
a container portion (56) that is adapted to contain a sample preservation reagent and the biopsy sample;
a proximal insertion portion (58a) connected detachably and liquid-tightly to an end portion of the sheath (36) at its proximal side and connected to the container portion (56) on its distal side, through which the catching member (42) is able to be removably inserted from an outside of the container (52) into the container portion (56); and
a distal insertion portion (58b) connected to the container portion (56) at its proximal side, through which the catching member (42) is able to removably project from the container portion (56) to an outside of the container (52) in a liquid-tight manner.

2. The biopsy device (10) according to claim 1, **characterized in that** the catching member (42) is able to be removably inserted through the proximal insertion portion (58a) in a liquid-tight manner.

3. The biopsy device (10) according to claim 1, **characterized in that** the proximal insertion portion (58a) connects an inner cavity of the sheath (36) to the container portion (56).

4. The biopsy device (10) according to one of claims 1 to 3, **characterized in that** the container portion (56) includes at least two sub-container portions (62a, 62b) divided from each other to store the sample preservation reagent and/or the biopsy sample.

5. The biopsy device (10) according to one of claims 1 to 3, **characterized in that** said at least two sub-container portions (62a, 62b) are connected in series between the proximal insertion portion (58a) and the distal insertion portion (58b) in such a manner that the catching member (42) is insertable through the first sub-container portion into the second sub-container portion, and
the catching member (42) is able to be removably inserted liquid-tightly through a connection portion (58c) provided between the sub-container portions (62a, 62b).

6. The biopsy device (10) according to one of claims 1 to 5, **characterized in that** the catching member (42) includes a puncture needle (42).

7. The biopsy device (10) according to one of claims 1 to 5, **characterized in that** the catching member (42) includes a forceps.

8. The biopsy device (10) according to one of claims 1 to 7, **characterized in that** the container (52) includes an identification tag (66).

9. The biopsy device (10) according to one of claims 1 to 8, **characterized in that** the sample preservation reagent includes a nucleic acid decomposition inhibiter reagent.

10. The biopsy device (10) according to one of claims 1 to 8, **characterized in that** the sample preservation reagent includes a cell preservation reagent.

11. The biopsy device (10) according to claim 4 or 5, **characterized in that** at least two of the sub-container portions (62a, 62b) contain different types of sample preservation reagents from each other.

## Patentansprüche

1. Biopsiegerät (10), aufweisend:
ein Auffangelement (42), das zum Auffangen und Freigeben einer Biopsieprobe eingerichtet ist;
einen Mantel (36), durch den das Auffangelement eingeführt werden kann; und
einen Behälter (52),
**dadurch gekennzeichnet, dass** der Behälter abnehmbar am distalen Ende des Mantels (36) angebracht ist, um die Biopsieprobe aufzubewahren;
wobei der Behälter (52) aufweist:
einen Behälterabschnitt (56), der dazu eingerichtet ist, ein Konservierungs-Reagens für die Probe und die Biopsieprobe aufzunehmen;
einen proximalen Einführabschnitt (58a), der an seiner proximalen Seite trennbar und flüssigkeitsdicht mit einem Endabschnitt des Mantels (36) und an seiner distalen Seite mit dem Behälterabschnitt (56) verbunden ist, durch den das Auffangelement (42) herausnehmbar von einer Außenseite des Behälters (52) in den Behälterabschnitt (56) eingeführt werden kann; und
einen distalen Einführabschnitt (58b), der an seiner proximalen Seite mit dem Behälterabschnitt (56) verbunden ist, durch den das Auffangelement (42) herausnehmbar vom Behälterabschnitt (56) zu einer Außenseite des Behälters (52) auf eine flüssigkeitsdichte Weise herausragen kann.

2. Biopsiegerät (10) nach Anspruch 1, **dadurch gekennzeichnet, dass** das Auffangelement (42) herausnehmbar durch den proximalen Einführabschnitt (58a) auf eine flüssigkeitsdichte Weise eingeführt werden kann.

3. Biopsiegerät (10) nach Anspruch 1, **dadurch gekennzeichnet, dass** der proximale Einführabschnitt (58a) einen inneren Hohlraum des Mantels (36) mit dem Behälterabschnitt (56) verbindet.

4. Biopsiegerät (10) nach einem der Ansprüche 1 bis 3, **dadurch gekennzeichnet, dass** der Behälterabschnitt (56) mindestens zwei Behälter-Unterabschnitte (62a, 62b) enthält, die voneinander getrennt sind, um das Konservierungs-Reagens für die Probe und/oder die Biopsieprobe aufzubewahren.

5. Biopsiegerät (10) nach einem der Ansprüche 1 bis 3, **dadurch gekennzeichnet, dass** die mindestens zwei Behälter-Unterabschnitte (62a, 62b) zwischen dem proximalen Einführabschnitt (58a) und dem distalen Einführabschnitt (58b) auf eine solche Weise in Reihe angeordnet sind, dass das Auffangelement (42) durch den ersten Behälter-Unterabschnitt in den zweiten Behälter-Unterabschnitt eingeführt werden kann, und
das Auffangelement (42) durch einen zwischen den Behälter-Unterabschnitten (62a, 62b) vorgesehenen Verbindungsabschnitt (58c) herausnehmbar und flüssigkeitsdicht eingeführt werden kann.

6. Biopsiegerät (10) nach einem der Ansprüche 1 bis 5, **dadurch gekennzeichnet, dass** das Auffangelement (42) eine Punkturnadel (42) enthält.

7. Biopsiegerät (10) nach einem der Ansprüche 1 bis 5, **dadurch gekennzeichnet, dass** das Auffangelement (42) eine Pinzette enthält.

8. Biopsiegerät (10) nach einem der Ansprüche 1 bis 7, **dadurch gekennzeichnet, dass** der Behälter (52) ein Kennzeichnungsetikett (66) enthält.

9. Biopsiegerät (10) nach einem der Ansprüche 1 bis 8, **dadurch gekennzeichnet, dass** das Konservierungs-Reagens für die Probe ein Zersetzungsinhibitorreagens für die Nukleinsäure enthält.

10. Biopsiegerät (10) nach einem der Ansprüche 1 bis 8, **dadurch gekennzeichnet, dass** das Konservierungs-Reagens für die Probe ein Zellkonservierungsreagens enthält.

11. Biopsiegerät (10) nach Anspruch 4 oder 5, **dadurch gekennzeichnet, dass** mindestens
zwei der Behälter-Unterabschnitte (62a, 62b) voneinander verschiedene Typen von Konservierungs-Reagentien für die Probe enthalten.

## Revendications

1. Dispositif de biopsie (10) comprenant :
- un élément de saisie (42) qui est destiné à saisir et relâcher un échantillon de biopsie ;
- une gaine (36) à l'intérieur de laquelle l'élément de saisie est insérable ; et
- un réservoir (52) **caractérisé en ce que** le réservoir est attaché de manière détachable à l'extrémité distale de la gaine (36) pour conserver l'échantillon de biopsie ;
dans lequel le réservoir (52) comprend :
- un partie de réservoir (56) qui est destinée à contenir un réactif de préservation de l'échantillon et de l'échantillon de biopsie ;
- une partie d'insertion proximale (58a) reliée de manière détachable et à étanchéité aux liquides à une partie d'extrémité de la gaine (36) sur son côté proximal et reliée à la partie de réservoir (56) sur son côté distal, à travers laquelle l'élément de saisie (42) peut être inséré de manière retirable à partir de l'extérieur du réservoir (52) à l'intérieur de la partie de réservoir (56) ;
- une partie d'insertion distale (58b) reliée à la partie de réservoir (56) sur son côté proximal, à travers laquelle l'élément de saisie (42) peut être en saillie par rapport à la partie de réservoir (56) vers l'extérieur du réservoir (52) à étanchéité aux liquides.

2. Dispositif de biopsie (10) selon la revendication 1, **caractérisé en ce que** l'élément de saisie (42) peut être inséré de manière retirable à travers la partie d'insertion proximale (58a) d'une manière étanche aux liquides.

3. Dispositif de biopsie (10) selon la revendication 1, **caractérisé en ce que** la partie d'insertion proximale (58a) relie une cavité interne de la gaine (36) à la partie de réservoir (56).

4. Dispositif de biopsie (10) selon l'une des revendications 1 à 3, **caractérisé en ce que** la partie de réservoir (56) inclut au moins deux sous parties de réservoir (62a, 62b) séparées l'une de l'autre pour conserver le réactif de préservation de l'échantillon et/ou l'échantillon de biopsie.

5. Dispositif de biopsie (10) selon l'une des revendications 1 à 3, **caractérisé en ce qu'**au moins deux sous parties de réservoir (62a, 62b) sont reliées en série entre la partie d'insertion proximale (58a) et la partie d'insertion distale (58b) de telle manière que l'élément de saisie (42) soit insérable à travers le premier sous partie de réservoir à l'intérieur de la seconde sous partie de réservoir, et l'élément de saisie (42) est capable d'être inséré et retiré à étanchéité aux liquides à travers la partie de liaison (58c) prévue entre les sous parties de réservoir (62a, 62b).

6. Dispositif de biopsie (10) selon l'une des revendications 1 à 5, **caractérisé en ce que** l'élément de saisie inclut une aiguille pour ponction (42).

7. Dispositif de biopsie (10) selon l'une des revendications 1 à 5, **caractérisé en ce que** l'élément de saisie (42) inclut un forceps.

8. Dispositif de biopsie (10) selon l'une des revendications 1 à 7, **caractérisé en ce que** le réservoir (52) inclut une étiquette d'identification (66).

9. Dispositif de biopsie (10) selon l'une des revendications 1 à 8, **caractérisé en ce que** le réactif de préservation de l'échantillon inclut un réactif d'inhibition de la décomposition de l'acide nucléique.

10. Dispositif de biopsie (10) selon l'une des revendications 1 à 8, **caractérisé en ce que** le réactif
de préservation de l'échantillon inclut un réactif de préservation de cellules.

11. Dispositif de biopsie (10) selon la revendication 4 ou 5, **caractérisé en ce qu'**au moins deux sous parties de réservoir (62a, 62b) comprennent des types de réactifs de préservation d'échantillon différents l'un de l'autre.
